# EUROPÄISCHE PATENTSCHRIFT

(11) **EP 1 146 924 B1**
(45) Veröffentlichungstag und Bekanntmachung des Hinweises auf die Patenterteilung: **01.06.2005**
(21) Anmeldenummer: 00900060.5
(22) Anmeldetag: 11.01.2000
(51) Int. Cl.: A61M 5/315

(54) **VORRICHTUNG ZUR VERABREICHUNG EINES INJIZIERBAREN PRODUKTS**
DEVICE FOR ADMINISTERING AN INJECTABLE PRODUCT
DISPOSITIF D'ADMINISTRATION D'UN PRODUIT INJECTABLE

(30) Priorität: 12.01.1999 DE 19900792
(43) Veröffentlichungstag der Anmeldung: 24.10.2001
(73) Patentinhaber: TecPharma Licensing AG, 3401 Burgdorf (CH)
(72) Erfinder: KIRCHHOFER, Fritz, CH-3454 Sumiswald (CH); GURTNER, Thomas, CH-3425 Koppigen (CH)
(86) Internationale Anmeldenummer: PCT/CH2000/000017
(87) Internationale Veröffentlichungsnummer: WO 2000/041753

(56) Entgegenhaltungen:
- WO-A-97/36625
- DE-A- 3 146 541
- DE-C- 19 723 647
- US-A- 5 226 895
- US-A- 5 454 793

## Beschreibung

Die Erfindung betrifft eine Vorrichtung zur Verabreichung eines injizierbaren Produkts nach dem Oberbegriff von Anspruch 1.

Ein Injektionsgerät, wie die Erfindung es auch betrifft, ist aus der WO 97/36625 bekannt. Das Injektionsgerät umfasst ein Gehäuse, ein Produktreservoir mit einem darin verschiebbar aufgenommenen Kolben, bei dessen Verschiebung in eine Vorschubrichtung Produkt aus dem Reservoir verdrängt wird, eine Antriebseinrichtung und eine Dosiereinrichtung.

Die Antriebseinrichtung umfasst ein Antriebsglied, das in die Vorschubrichtung bis in eine proximale Endposition und gegen die Vorschubrichtung bis in eine distale Endposition entlang einer Verschiebeachse verschiebbar ist, und ein Abtriebsglied, das an einer Verschiebung gegen die Vorschubrichtung gehindert ist, bei einer Verschiebung des Antriebsglieds in Vorschubrichtung jedoch von dem Antriebsglied mitgenommen wird und dabei den Kolben in Vorschubrichtung schiebt, so dass Produkt aus dem Reservoir verdrängt wird. Bei einer Einstellung der bei einem vollen Hub von dem Abtriebsglied zurücklegbaren Weglänge und damit der ausschüttbaren Produktdosis wird das Antriebsglied gegen die Vorschubrichtung und relativ zum Abtriebsglied in seine distale Endposition zurück verschoben. Die distale Endposition wird mittels einer Dosiereinrichtung eingestellt, während die proximale Endposition durch einen Anschlag an dem Gehäuse definiert wird.

Die Dosiereinrichtung umfasst das Antriebsglied und das Dosierglied zur Einstellung der distalen Endposition des Antriebsglieds. Das Dosierglied ist um die Verschiebeachse des Antriebsglieds verdrehbar in dem Gehäuse gelagert. Es weist einen um diese Verschiebeachse spiralig umlaufenden Dosieranschlag auf, an den das Antriebsglied bei einer Verschiebung in die distale Position anschlägt, d.h. die Drehwinkelposition des Dosierglieds bestimmt die distale Position des Antriebsglieds.

Die ausschüttbare Produktdosis wird durch Verdrehen des Dosierglieds in diskreten Schritten gewählt. Hierfür verrastet das Dosierglied in regelmäßig zwischen dem Gehäuse und dem Dosierglied gebildeten Drehwinkelrastpositionen. Eine Verdrehung des Dosierglieds zwischen zwei benachbarten Rastpositionen entspricht einer einstellbaren, kleinsten Produktdosis. Der spiralig umlaufende Dosieranschlag des Dosierglieds weist einen diskontinuierlichen Verlauf auf. Er fällt von einem proximalsten Abschnitt in diskreten Schritten bis zu seinem distalsten Abschnitt ab. An dem Antriebsglied ist als Dosiergegenanschlag ein davon radial abragender Dosiernocken ausgebildet, der bei einer Zurückverschiebung des Antriebsglieds zum Dosieren bis gegen den durch die Verdrehung des Dosierglieds ihm gegenüberliegenden Abschnitt des Dosieranschlags des Dosierglieds geschoben wird. Der Dosiernocken des Antriebsglieds ist bei dieser Konstruktion sehr schmal.

Die Erfindung hat es sich zur Aufgabe gemacht, eine Vorrichtung zur Verabreichung eines injizierbaren Produkts, insbesondere der vorgenannten Art, zu schaffen, die eine hohe mechanische Festigkeit in Bezug auf die zur Produktdosierung dienenden Komponenten aufweist, wobei die Feinheit und Exaktheit der Dosierung der vorbekannten Vorrichtung zumindest beibehalten werden soll.

Diese Aufgabe wird durch den Gegenstand von Anspruch 1 gelöst, indem der spiralige Dosieranschlag einen kontinuierlichen Verlauf mit einer konstanten Steigung relativ zur Verschiebeachse des Antriebsglieds aufweist.

Obgleich der spiralige Dosieranschlag grundsätzlich nicht vollständig um die Verschiebeachse umlaufen muß, wird ein vollständig umlaufender Dosieranschlag bevorzugt. Der spiralige Dosieranschlag ist bevorzugt am Dosierglied ausgebildet und ein Dosiergegenanschlag dazu an dem Antriebsglied. Grundsätzlich kann die Anordnung aber auch vertauscht sein.

Beschädigungen des spiraligen Dosieranschlags durch allzu kraftvolles Zurückschieben des Antriebsglieds können nicht auftreten. Insbesondere wird die Gefahr von Beschädigungen des spiraligen Dosieranschlags wegen des Wegfalls von Kanten reduziert. Ferner wird die Herstellung der vorzugsweise durch ein Spritzgußteil aus Kunststoff gebildeten Komponente mit dem spiraligen Dosieranschlag vereinfacht. Es kann auch die Dosierung verfeinert werden, da ein mindestens erforderlicher Winkelabstand zwischen benachbarten, diskret festgelegten oder festlegbaren Drehwinkelpositionen des Dosierglieds allenfalls noch von vorzugsweise vorgesehenen Mitteln zur Festlegung dieser Drehwinkelpositionen bestimmt wird, die bevorzugt durch Rastmittel eines Rastmechanismus zwischen dem Dosierglied und dem Gehäuse der Vorrichtung gebildet werden.

Aufgrund der Erfindung kann der Dosiergegenanschlag, der vorzugsweise an dem Antriebsglied ausgebildet ist, eine quer zur Vorschubrichtung gemessene Breite aufweisen, die größer ist als ein in Drehrichtung gemessener Abstand zwischen zwei unmittelbar aufeinander folgenden Drehwinkelpositionen des Dosierglieds. Mit anderen Worten: Es kann der Winkel, über den sich der Dosiergegenanschlag zu dem spiraligen Dosieranschlag erstreckt, größer sein als der Winkel zwischen zwei unmittelbar aufeinander folgenden, festgelegten oder festlegbaren Drehwinkelpositionen des Dosierglieds. Besonders bevorzugt beträgt die Breite des Dosiergegenanschlags wenigstens das Zweifache des Winkelabstands zwischen zwei unmittelbar aufeinander folgenden Drehwinkelpositionen des Dosierglieds. Es kann bei gleicher Feinheit und Exaktheit der Dosierung, wie beispielsweise bei dem Injektionsgerät der WO 97/36625, der Dosiergegenanschlag quer zur Vorschubrichtung erheblich verbreitert werden. Maximal kann er vollständig um die Verschiebeachse umlaufend ausgebildet sein. Vorzugsweise beträgt sein Erstreckungswinkel jedoch nicht mehr als das Zehnfache des vorgenannten Winkelabstands.

Das Antriebsglied und das Dosierglied sind vorzugsweise so angeordnet, dass das eine das andere umgibt. Entsprechend können die beiden zur Dosierung zusammenwirkenden Dosieranschläge, nämlich derjenige des Antriebsglieds und derjenige des Dosierglieds, an den einander zugewandt gegenüberliegenden Mantelflächen des Antriebsglieds und des Dosierglieds ausgebildet sein. Bevorzugt umgibt das Dosierglied einen distalen Bereich des Antriebsglieds konzentrisch. Bei dieser Ausbildung werden nach einem bevorzugten Ausführungsbeispiel der spiralige Dosieranschlag des Dosierglieds durch die proximale Stirnseite des Dosierglieds und der Dosieranschlag des Antriebsglieds durch einen vom Außenmantel des Antriebsglieds quer zur Vorschubrichrung abragenden Dosiemocken gebildet.

Ein bevorzugtes Ausführungsbeispiel der Erfindung wird nachfolgend anhand von Figuren erläutert. Es zeigen:
- Fig. 1: eine Injektionsvorrichtung mit einer erfindungsgemäßen Dosiereinrichtung in einem Längsschnitt und
- Fig. 2: eine erfindungsgemäßes Dosierglied in einer Ansicht.

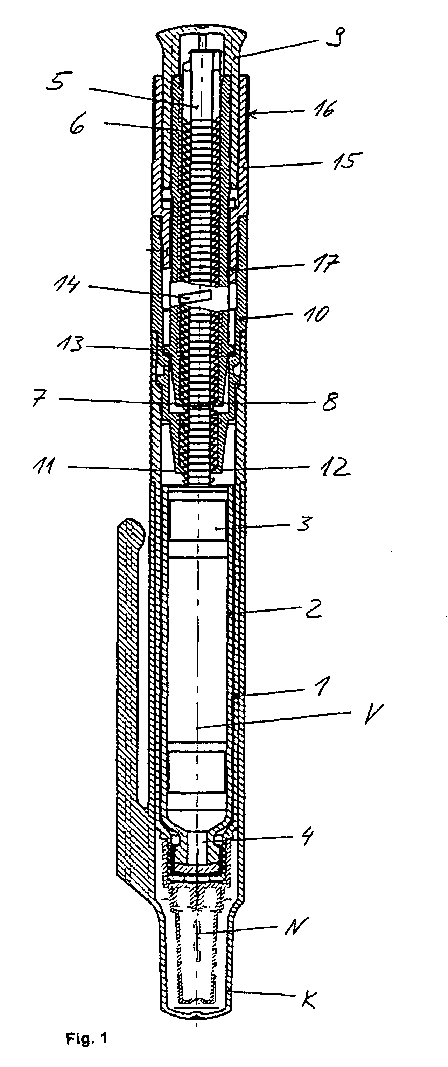

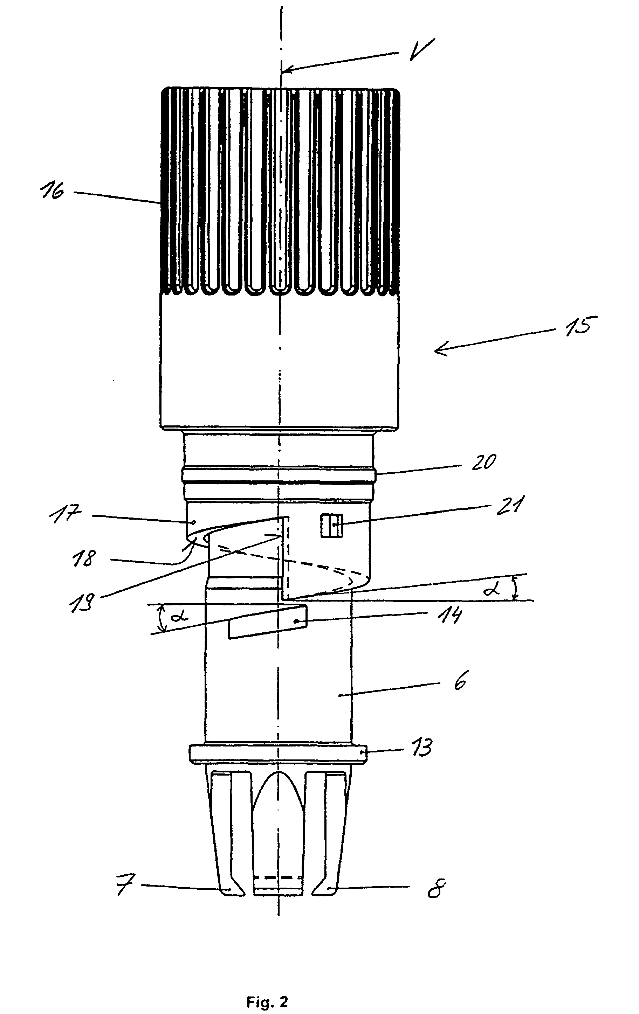

Figur 1 zeigt ein Injektionsgerät, im Ausführungsbeispiel ein Injektionspen, in einem Längsschnitt. Die Figur 2 zeigt im Detail ein Antriebsglied 6 und ein Dosierglied 15 wie sie in dem Injektionsgeräts auch angeordnet sind.

Das Injektionsgerät weist ein Gehäuse mit einer vorderen Gehäusehülse 1 und einer damit fest verbundenen hinteren Gehäusehülse 10 auf. Die vordere Gehäusehülse 1 dient als Aufnahme für eine Ampulle 2. In der Ampulle 2 ist ein flüssiges Produkt in Form einer Wirkstofflösung, beispielsweise Insulin, enthalten. Ferner ist in der Ampulle 2 ein Kolben 3 aufgenommen. Durch Verschiebung des Kolbens 3 in Vorschubrichtung auf einen Ampullenauslass 4 zu wird das Produkt aus der Ampulle 2 durch deren Auslass 4 hindurch verdrängt und durch eine Injektionsnadel N ausgeschüttet. Die vordere Gehäusehülse 1 ist durch eine Kappe K geschützt. Die Nadel N ist durch eine Nadelkappe nochmals geschützt.

Die Verschiebung des Kolbens 3 in Vorschubrichtung wird durch eine Antriebseinrichtung bewirkt, die in der hinteren Gehäusehülse 10 aufgenommen ist. Die Antriebseinrichtung umfasst als Abtriebsglied eine Zahnstange 5, die unmittelbar auf den Kolben 3 wirkt, und ein Antriebsglied 6. Das Antriebsglied 6 ist in der hinteren Gehäusehülse 10 in und gegen die Vorschubrichtung des Kolbens 3 entlang einer Verschiebeachse V geradverschiebbar gelagert. Ein Deckel 9, der mit dem Antriebsglied 6 verschiebe- und verdrehgesichert verbunden ist, ragt aus dem Gehäuse nach hinten heraus.

Ein als Hülsenkörper ausgebildetes Dosierglied 15 ist mit der hinteren Gehäusehülse 10 verschiebegesichert, jedoch um die gemeinsame Längsachse, die mit der Verschiebeachse V zusammenfällt, verdrehbar verbunden. Das Dosierglied 15 ragt mit einem vorderen Hülsenteil 17 in die hintere Gehäusehülse 10. Sein hinterer Hülsenteil ragt aus der hinteren Gehäusehülse 10 heraus. Wie am besten aus der Zusammenschau der Figuren 1 und 2 zu erkennen ist, dient zur verschiebesicheren Befestigung des Dosierglieds 15 ein an dem vorderen Hülsenteil 17 ausgebildeter Ringwulst 20, der in einer an dem Innenmantel der hinteren Gehäusehülse 10 umlaufenden Ausnehmung eingeschnappt ist. Das hintere Hülsenteil des Dosierglieds 15 ist mit einer Profilierung 16 versehen, um das Dosierglied 15 griffsicher manuell verdrehen zu können.

Vor der Profilierung 16 trägt das Dosierglied 15 um seine äußere Mantelfläche umlaufend eine gut sichtbare Dosisskala, die auf festgelegte Drehwinkelpositionen, in denen das Dosierglied 15 gegen die hintere Gehäusehülse 10 verrastet, abgestimmt ist. Der Rastmechanismus zwischen dem Dosierglied 15 und der hinteren Gehäusehülse 10 wird durch Erhebungen 21 an dem Außenmantel des vorderen Hülsenteils 17 des Dosierglieds 15 und Vertiefungen in dem Innenmantel der hinteren Gehäusehülse 10 gebildet. Die Vertiefungen sind in gleichen Winkelabständen nebeneinander auf gleicher Höhe umlaufend an dem Innenmantel der hinteren Gehäusehülse 10 angeordnet. In den fixen Drehwinkelrastpositionen des Dosierglieds 15 werden die mehreren Erhebungen 21 exakt in den jeweils gegenüberliegenden Vertiefungen in dem Innenmantel der hinteren Gehäusehülse 10 aufgenommen.

Im komplett montierten Zustand des Injektionsgeräts, wie es in Figur 1 dargestellt ist, wird das Dosierglied 15 von dem Antriebsglied 6 durchragt. Das Dosierglied 15 umgibt einen distalen Teil des Antriebsglieds 6 und auch des Abtriebsglieds 5 konzentrisch. Der Deckel 9 ragt mit einem Hülsenteil in einen zwischen dem Antriebsglied 6 und dem Dosierglied 15 gebildeten Ringspalt hinein. Auch der Deckel 9 trägt in seinem aus dem Dosierglied 15 herausragenden Mantelbereich eine Markierung, die im Zusammenwirken mit der Markierung des Dosierglieds 15 auch nach mehreren vollen Drehungen des Dosierglieds 15 die genaue Bestimmung der aus der Ampulle 2 insgesamt verabreichten Produktmenge ermöglicht.

Durch Verdrehen des Dosierglieds 15 wird die in Vorschubrichtung von dem Antriebsglied 6 und der Zahnstange 5 maximal zurücklegbare Dosisweglänge eingestellt und damit auch die bei einer Injektion maximal ausschüttbare Produktdosis. Hierfür ist der vordere Hülsenteil 17 des Dosierglieds 15 an seiner vorderen, proximalen Stirnseite 18 spiralig umlaufend ausgebildet, d. h. der vordere Hülsenteil 17 fällt in Bezug auf die Verschiebeachse V des Antriebsglieds 6 von einem vordersten Stirnseitenabschnitt in eine Umfangsrichtung fortschreitend ab.

Die Dosierung erfolgt in einer in Bezug auf die Vorschubrichtung vordersten, proximalen Endposition des Antriebsglieds 6, in der ein von der äußeren Mantelfläche des Antriebsgliebs 6 radial abstehender Anschlagnocken oder -kragen 13 an einem durch die hintere Gehäusehülse 10 gebildeten Anschlag anliegt. In dieser proximalen Endposition des Antriebsglieds 6 wird das Dosierglied 15 um die Verschiebeachse V relativ zur hinteren Gehäusehülse 10 verdreht, bis es die gewünschte Dosier bzw. Drehwinkelrastposition erreicht hat. In dieser Dosierposition verbleibt zwischen einem ebenfalls von der äußeren Mantelfläche des Antriebsglieds 6 abragenden weiteren Kragen bzw. Nocken, der einen Dosieranschlag 14 bildet und daher im weiteren Dosiernocken 14 genannt wird, und der diesem Dosiernocken 14 gegenüberliegenden, proximalen Stirnseite 18 des Dosierglieds 15 ein lichter Dosierabstand. Um den Dosierabstand kann das Antriebsglied 6 relativ zur hinteren Gehäusehülse 10 und damit auch relativ zum Kolben 3 gegen die Vorschubrichtung zurückgezogen werden. Das Zurückziehen erfolgt manuell durch Ziehen an dem Deckel 9. Der Dosierabstand ist gleich der Dosisweglänge bei der nachfolgenden Verabreichung.

Bei einem Zurückverschieben bzw. Zurückziehen des Antriebsglieds 6 verbleibt die Zahnstange 5 in ihrer bei dem Dosiervorgang eingenommenen Verschiebelage relativ zum Gehäuse. Sie wird durch an der hinteren Gehäusehülse 10 ausgebildete Sperrmittel 11 und 12 gegen eine Verschiebung gegen die Vorschubrichtung gesichert. Die Sperrmittel 11 und 12 sind Rastnocken, die je an einem vorderen Ende einer elastisch nachgiebigen Zunge ausgebildet sind und von ihrer Zunge radial nach innen auf die Zahnstange 5 zu ragen. Die Sperrmittel 11 und 12 wirken je mit einer ihnen zugewandten Zahnreihe der Zahnstange 5 zusammen, derart, dass sie eine Verschiebung der Zahnstange 5 in Vorschubrichtung zulassen und eine Verschiebung gegen die Vorschubrichtung durch formschlüssigen Sperreingriff verhindern.

Das Zusammenwirken des Antriebsglieds 6 und des Dosierglieds 15 zum Zwecke der Dosierung ist am besten in Figur 2 erkennbar. Figur 2 zeigt das Antriebsglied 6 unmittelbar vor Erreichen seiner distalen Endposition, d.h. der Anschlagposition an dem Dosierglied 15. Die beiden hierfür zusammenwirkenden Dosieranschläge, nämlich die proximale, spiralig umlaufende Stirnseite 18 des Dosierglieds 15 und der quer von dem Antriebsglied 6 abragende Dosiernocken 14, weisen relativ zu der Verschiebeachse V die gleiche konstante Steigung bzw. den gleichen konstanten Steigungswinkel α auf. Für den spiraligen Dosieranschlag 18 ergibt dies den erkennbaren, stetigen Verlauf mit der konstanten Steigung α. Es entsteht eine einzige, parallel zur Verschiebeachse V sich erstreckende Kante 19, die die proximale Spitze des Dosieranschlags 18 mit seinem distalen Grund verbindet. Weitere Kanten weist die proximale Stirnseite 18 des Dosierglieds 15 nicht auf. Der durch den Dosiemocken gebildete Dosieranschlag 14 ist zumindest an seiner dem spiraligen Dosieranschlag 18 zugewandt gegenüberliegenden Fläche derjenigen des spiraligen Dosieranschlags 18 angepasst, derart, dass der Dosiernocken 14 in der in Figur 2 dargestellten Anordnung aus Antriebs- und Dosierglied auf dem Dosieranschlag 18 bei einer 360°-Drehung wie auf einer schiefen Ebene entlang geschoben werden kann. Die Dosierschritte des Injektionsgeräts werden ausschließlich durch den Rastmechanismus zwischen der hinteren Gehäusehülse 10 und dem Dosierglied 15 definiert. Der den Dosieranschlag 14 bildende Dosiemocken des Antriebsglieds 6 kann von der Feinheit der Dosierschritte unabhängig in einer in Bezug auf seine mechanische Festigkeit optimalen Weise gestaltet werden. Im Ausführungsbeispiels erstreckt sich der Dosieranschlag 14 an dem Außenmantel des Antriebsglieds 6 über einen Winkel, der etwa fünf Mal so groß ist, wie der Winkelabstand zwischen zwei unmmittelbar aufeinanderfolgenden Drehwinkelrastpositionen des Dosierglieds 15.

Die Zahnstange 5 wird durch einen im Querschnitt rechteckigen Stangenkörper gebildet, der in einem in Bezug auf die Vorschubrichtung vorderen Bereich an allen vier Seiten mit je einer Sägezahnreihe versehen ist. In Figur 2 sind zwei an gegenüberliegenden Seiten der Zahnstange 5, den Spetrmitteln 11 und 12 gegenüberliegend ausgebildete Zahnreihen zu erkennen. Zusätzlich zu diesen beiden Zahnreichen weist die Zahnstange 5 zwei weitere, an gegenüberliegenden Seitenflächen der Zahnstange 5 ausgebildete Sägezahnreihen auf. Die einzelnen Zähne jeder der Zahnreihen der Zahnstange 5 sind jeweils in Vorschubrichtung verjüngt ausgebildet; im Ausführungsbeispiel sind die Zahnflanken einfach plan und schräg. Der Rücken jedes Zahns ist einfach plan und weist senkrecht zur Vorschubrichtung und damit zur Längsrichtung des Injektionsgeräts und der Zahnstange 5. Mit 5a sind jeweils die regelmäßigen bzw. regulären Zahnlücken der Zahnreihen bezeichnet. Die vier Zahnreihen weisen die gleiche Zahnteilung auf und sind auf gleichen Höhen um die Zahnstange 5 ausgebildet.

Die Zahnreihen sind innerhalb einer Zahnteilung zueinander in einem Versatz in Bezug auf die Vorschubrichtung angeordnet.

Die Sperrmittel 11 und 12 und zwei weitere Sperrmittel, die mit den jeweils zugewandten weiteren Zahnreihen zusammenwirken, befinden sich in Bezug auf die Vorschubrichtung auf gleicher Höhe in jeweils 90° Winkelabstand. Wegen des Zahnreihenversatzes greift stets nur eines der Sperrmittel mit einem tiefsten Zahneingriff in eine Zahnlücke der ihm zugewandten Zahnreihe, wenn die Zahnstange 5 vorgeschoben wird. Den drei anderen Sperrmitteln liegen jeweils Flanken von Zähnen der ihnen zugewandten Zahnreihen gegenüber, so dass diese anderen Sperrmittel von der Zahnstange 5 weggebogen werden. Bei einer Verschiebung der Zahnstange 5 in Vorschubrichtung kommen auf diese Weise die Sperrmittel sukzessive in tiefstmöglichen Eingriff mit der ihnen jeweils zugewandten Zahnreihe; es ergibt sich insgesamt ein alternierender Eingriff der Sperrmittel. Das jeweils in einen Zahngrund oder zu einem Zahngrund hin elastisch voll eingeschnappte Sperrmittet, sperrt die Zahnstange 5 gegen eine Verschiebung gegen die Vorschubrichtung.

Die Verschiebung der Zahnstange 5 in Vorschubrichtung wird von dem Antriebsglied 6 bewirkt. Hierfür läuft das Antriebsglied 6 in Vorschubrichtung in vier Zungen aus, die an ihren vorderen Enden radial nach innen abragende Rastnocken tragen. Von den derart gebildeten Mitnehmern sind die beiden sich gegenüberliegenden Mitnehmer 7 und 8 in Figur 1 dargestellt. Im Ausführungsbeispiel sind die Mitnehmer und die Sperrmittel in ihrer Form und Funktionsweise gleich. Beide werden durch Rastnocken an elastisch nachgiebigen Zungen gebildet. Bei einer Verschiebung des Antriebsglieds 6 in Vorschubrichtung stemmt sich jeweils einer der Mitnehmer gegen den Rücken eines der Zähne der ihm zugewandten Zahnreihe und bewirkt so die zwangsweise Mitnahme der Zahnstange 5 in Vorschubrichtung. Aufgrund ihrer elastischen Nachgiebigkeit und der Vorwärtspfeilung der Zähne gleiten die Mitnehmer bei einer Verschiebung des Antriebsglieds 6 gegen die Vorschubrichtung über die Zahnreihen der durch die Sperrmittel gesperrten Zahnstange 5. Da die Mitnehmer auf gleicher Höhe in Bezug auf die Vorschubrichtung in Rastnocken auslaufen, greifen auch niemals zwei der Mitnehmer mit tiefstem Zahneingriff gleichzeitig in eine der regulären Zahnlücken der Zahnstange 5 ein.

In Figur 1 ist das Injektionsgerät in einer Ausgangsstellung dargestellt, in der die Zahnstange 5 ihre hinterste, proximale Endposition relativ zu der hinteren Gehäusehülse 10 und auch relativ zum Antriebsglied 6 einnimmt. In dieser Ausgangsstellung wird die hintere Gehäusehälfte komplett montiert mit Zahnstange 5 und Antriebsglied 6 einschließlich Deckel 9 und Dosierglied 15 herstellerseitig geliefert. Die Ausgangsstellung entspricht somit der Lagerstellung des Injektionsgeräts, insbesondere der Antriebs- und Dosiereinrichtung des Injektionsgeräts. Im Ausführungsbeispiel ist das Injektionsgerät ein Einwegpen. Eine Wiederverwendbarkeit, d.h. ein Ampullenaustausch, kann jedoch mit einfachen Modifikationen erreicht werden.

In der Ausgangsstellung des Injektionsgeräts mit eingesetzter Ampulle 2 wird die mit der ersten Injektion zu verabreichende Produktdosis vom Benutzer eingestellt. Hierzu wird das Dosierglied 15 in eine bestimmte Dosierposition gedreht, die der gewünschten Produktdosis entspricht. In dieser Dosierposition weist der Dosiemocken 14 des Antriebsglieds 6 zu dem ihm gegenüberliegenden Dosieranschlag 18, gebildet von der proximalen Stirnseite des Dosierglieds 15, den lichten Dosierabstand auf. Nur das Sperrmittel 11 liegt in der Ausgangsstellung an einem Zahnrücken der Zahnstange 5 auf Sperranschlag. Die anderen Sperrmittel sind zwar bis in ihre entlasteten Neutralstellungen zur Zahnstange 5 hin vorgeschnappt, sie kommen in der Ausgangsstellung jedoch in Zahnlücken zu liegen, die gegenüber den regulären Zahnlücken verlängert sind. Von den Mitnehmern liegt in der Ausgangsstellung nur der Mitnehmer 7 auf Anschlag zu einem Zahnrücken. Die anderen Mitnehmer liegen in der Ausgangsstellung entlastet in ihren Neutralstellungen in den ihnen zugewandten Zahnlücken, d.h. sie werden in der Ausgangsstellung nicht weggebogen. Vor ihren verlängerten Zahnlücken bei den Sperrmitteln weisen die Zahnreihen der Zahnstange 5 je einen Zahn auf. Diese Zähne, die die verlängerten Zahnlücken in Vorschubrichtung begrenzen, dienen lediglich einem Funktionstest des Injektionsgeräts. Unmittelbar nach dem Zusammenbau des Geräts wird die Zahnstange 5 durch die konzentrisch zu ihr angeordneten Sperrmittel hindurch bis in die gezeigte Ausgangsstellung gedrückt.

Das Antriebsglied 6 wird durch Ziehen an dem Deckel 9 aus seiner proximalen Endposition in Bezug auf die hintere Gehäusehülse 10 gegen die Vorschubrichtung bis in die distale Endposition zurückgezogen. Bei dem Zurückziehen des Antriebsglieds 6 gleiten dessen Mitnehmer über die ihnen zugewandten Zahnreihen der Zahnstange 5, die an einer Mitnahme durch das Sperrmittel 11 gehindert wird.

Bei der Injektion werden das Antriebsglied 6 und damit auch die Zahnstange 5 in Vorschubrichtung durch Drücken gegen den Deckel 9 um die Dosisweglänge verschoben. Dabei drückt die Zahnstange 5 den Kolben 3 in der Ampulle 2 auf den Auslass 4 zu, und es wird Produkt ausgeschüttet.

Im Ausführungsbeispiel sind die Mitnehmer des Antriebsglieds 6 in Bezug auf die Vorschubrichtung hinter den Sperrmitteln angeordnet. Die konzentrische Anordnung der Sperrmittel und der Mitnehmer ist so gestaltet, dass sie entsprechend der Zahnform der Zahnreihen der Zahnstange 5 gegen ihre eigenen elastischen Rückstellkräfte radial nach außen von der Zahnstange 5 weggebogen werden können. Im Ausführungsbeispiel liegen jeweils die Spettmittel unter sich und jeweils die Mitnehmer unter sich auf gleicher Höhe in Bezug auf die Vorschubrichtung, während die Zahnreihen der Zahnstangen 5 solch einen Versatz zueinander aufweisen, dass die regulären Zahnlücken der Zahnreihen auf unterschiedlichen Höhen in Bezug auf die Vorschubrichtung zu liegen kommen. Hierdurch wird bewirkt, dass niemals mehr als ein Sperrmittel bzw. ein Mitnehmer in eine der regulären Zahnlücken eingreift. Statt dieser Anordnung können auch die Sperrmittel und auch die Mitnehmer in Bezug auf die Vorschubrichtung entsprechend auf unterschiedlichen Höhen versetzt und die Zahnreihen der Zahnstange 5 auf gleicher Höhe angeordnet sein. Die im Ausführungsbeispiel gewählte Anordnung hat jedoch fertigungstechnische Vorteile.

In der in den Figuren dargestellten Ausgangsstellung, die insbesondere für die in der hinteren Gehäusehülse 10 aufgenommenen Teile des Injektionsgeräts, nämlich die Zahnstange 5, das Antriebsglied 6 und die Sperrmittel auch die Lagerstellung ist, würde die Gefahr einer Materialermüdung bei solchen Sperrmitteln und Mitnehmern bestehen, die in der Ausgangsstellung nicht in Zahnlücken so einschnappen können, dass sie zumindest teilweise oder, wie im Ausführungsbeispiel, vollkommen entlastet sind. Diese Sperrmittel und Mitnehmer wären nämlich in der Ausgangsstellung abgebogen. In der abgebogenen Stellung sind die Mitnehmer und Sperrmittel elastisch vorgespannt. Hält dieser Zustand über längere Zeiten an, so kann ein elastisches Rückbiegen bis in die Funktionsstellung, nämlich der Anschlagstellung gegen einen Zahnrücken, nicht mit der erforderlichen Sicherheit gewährleistet werden.

Die Zahnstange 5 weist jedoch verlängerte Zahnlücken dort auf, wo in der Ausgangsstellung des Injektionsgeräts Sperrmittel und Mitnehmer eingreifen, die in der Ausgangsstellung nicht auf Anschlag zu Zahnrücken der Zahnstange 5 sind.

In der Ausgangsstellung blockiert das Sperrmittel 11 die Zahnstange 5 gegen eine Verschiebung gegen die Vorschubrichtung. In dieser Ausgangsstellung wird zunächst die mit der nächsten Injektion zu verabreichende Produktdosis mit dem Dosierglied 15 gewählt. Anschließend wird das Antriebsglied 6 um den dieser Dosis entsprechenden Dosierabstand zurückgezogen. Dabei gleiten die Mitnehmer über die Zähne der ihnen jeweils zugewandten Zahnreihe der Zahnstange 5, wobei durch den Versatz der Zahnreihen sichergestellt wird, dass die Mitnehmer sukzessive in einem regelmäßigen Wechsel einschnappen, wodurch gegenüber nur einem einzigen Mitnehmer innerhalb einer Zahnteilung mehrere Rastvorgänge stattfinden. In der vom Dosierglied 15 vorgegebenen distalen Endposition des Antriebsglieds 6 wird ein Einschnappen wenigstens eines der Mitnehmer weit sicherer gewährleistet als dies bei nur einer Zahnreihe und einem Mitnehmer der Fall wäre. Sinngemäß das gleiche gilt für das Zusammenwirken der Zahnreihen und der Sperrmittel. Bei einer Verschiebung des Antriebsglieds 6 gegen die Vorschubrichtung und auch bei einer Verschiebung der Zahnstange 5 in Vorschubrichtung gelangen einer der Mitnehmer und eines der Sperrmittel je als nächstes in tiefsten Zahneingriff und somit in Mitnahmeeingriff bzw. Sperreingriff, die in der Ausgangsstellung je in eine verlängerte Zahnlücke einschnappen.

## Patentansprüche

1. Vorrichtung zur dosierten Verabreichung eines injizierbaren Produkts, die Vorrichtung umfassend:
a) ein Gehäuse (1), mit einem Reservoir (2) für das Produkt,
b) einen Kolben (3), der bei einer Verschiebung in eine Vorschubrichtung auf einen Reservoirauslass (4) zu Produkt aus dem Reservoir (2) verdrängt,
c) ein Antriebsglied (6), das in die Vorschubrichtung bis in eine proximale Endposition und gegen die Vorschubrichtung bis in eine distale Endposition entlang einer Verschiebeachse (V) verschiebbar ist,
d) ein Abtriebsglied (5), das an einer Verschiebung gegen die Vorschubrichtung gehindert ist und bei einer Verschiebung des Antriebsglieds (6) in Vorschubrichtung von dem Antriebsglied (6) mitgenommen wird und dabei den Kolben (3) in Vorschubrichtung schiebt,
e) ein Dosierglied (15), das zur Einstellung einer bei einer Verabreichung ausschüttbaren Produktdosis um die Verschiebeachse (V) des Antriebsglieds (6) drehbar ist,
f) wobei in der distalen Endposition das Antriebsglied (6) und das Dosierglied (15) je mit wenigstens einem Dosieranschlag (14, 18) von denen der eine an dem Antriebsglied (6) und der andere an dem Dosierglied (15) ausgebildet ist, auf Anschlag zu liegen kommen und wobei
g) wenigstens einer dieser Dosieranschläge (14, 18) um die Verschiebeachse (V) des Antriebsglieds (6) spiralig zumindest teilweise umläuft,
**dadurch gekennzeichnet, dass**
h) der wenigstens eine spiralige Dosieranschlag (18) einen kontinuierlichen Verlauf mit einer konstanten Steigung (α) aufweist.

2. Vorrichtung nach Anspruch 1, **dadurch gekennzeichnet, dass** das Dosierglied (15) zwischen festgelegten oder festlegbaren Drehwinkelpositionen verdrehbar ist, vorzugsweise indem es in Drehwinkelrastpositionen verrastet, und dass der vorzugsweise an dem Antriebsglied (6) ausgebildete andere Dosieranschlag (14) zu dem vorzugsweise an dem Dosierglied (15) ausgebildeten spiraligen Dosieranschlag (18) sich über einen Winkel erstreckt, der größer ist als ein Winkelabstand zwischen unmittelbar aufeinander folgenden Drehwinkelpositionen des Dosierglieds (15).

3. Vorrichtung nach dem vorhergehenden Anspruch, **dadurch gekennzeichnet, dass** der andere Dosieranschlag (14) sich über einen Winkel erstreckt, der das wenigstens Zweifache des Winkelabstands zwischen unmittelbar aufeinander folgenden Drehwinkelpositionen des Dosierglieds (15) beträgt.

4. Vorrichtung nach Anspruch 2 oder 3, **dadurch gekennzeichnet, dass** der andere Dosieranschlag (14) sich über einen Winkel erstreckt, der das höchstens Zehnfache des Winkelabstands zwischen unmittelbar aufeinander folgenden Drehwinkelpositionen des Dosierglieds (15) beträgt.

5. Vorrichtung nach einem der vorhergehenden Ansprüche, **dadurch gekennzeichnet, dass** der vorzugsweise an dem Antriebsglied (6) ausgebildete andere Dosieranschlag (14) zu dem vorzugsweise an dem Dosierglied (15) ausgebildeten spiraligen Dosieranschlag (18) die gleiche Steigung (α) aufweist wie der spiralige Dosieranschlag (18).

6. Vorrichtung nach dem vorhergehenden Anspruch, **dadurch gekennzeichnet, dass** der spiralige Dosieranschlag (18) an einer proximalen Stirnseite des Dosierglieds (15) und der andere Dosiergegenanschlag (14) durch einen quer zur Vorschubrichtung von dem Antriebsglied (6) abragenden Nocken gebildet wird.

## Claims

1. A device for the metered administration of an injectable product, the device comprising:
a) a housing (1) having a reservoir (2) for the product,
b) a plunger (3) which upon displacement in a feed direction towards a reservoir outlet (4) displaces product out of the reservoir (2),
c) a drive member (6) which is displaceable in the feed direction into a proximal end position and in opposite relationship to the feed direction into a distal end position along a displacement axis (V),
d) a driven member (5) which is prevented from being displaced in opposite relationship to the feed direction and upon displacement of the drive member (6) in the feed direction is entrained by the drive member (6) and in that case displaces the plunger (3) in the feed direction,
e) a metering member (15) which is rotatable about the displacement axis (V) of the drive member (6) for adjusting a product dose which can be expelled in an administration operation,
f) wherein in the distal end position the drive member (6) and the metering member (15) come into contact with at least one respective metering abutment (14, 18) of which one is provided on the drive member (6) and the other on the metering member (15), and wherein
g) at least one of said metering abutments (14, 18) at least partially extends in a spiral around the displacement axis (V) of the drive member (6),
**characterised in that**
h) the at least one spiral metering abutment (18) is of a continuous configuration with a constant pitch (α).

2. A device according to claim 1 **characterised in that** the metering member (15) is rotatable between fixed or fixable rotary angular positions, preferably by latching in rotary angular positions, and that the other metering abutment (14) which is preferably provided on the drive member (6) extends relative to the spiral metering abutment (18) which is preferably provided on the metering member (15) over an angle which is greater than an angular spacing between directly successive rotary angular positions of the metering member (15).

3. A device according to the preceding claim **characterised in that** the other metering abutment (14) extends over an angle which is at least double the angular spacing between directly successive rotary angular positions of the metering member (15).

4. A device according to claim 2 or claim 3 **characterised in that** the other metering abutment (14) extends over an angle which is at most ten times the angular spacing between directly successive rotary angular positions of the metering member (15).

5. A device according to one of the preceding claims **characterised in that** the other metering abutment (14) which is preferably provided on the drive member (6), relative to the spiral metering abutment (18) which is preferably provided on the metering member (15), is of the same pitch (α) as the spiral metering abutment.

6. A device according to the preceding claim **characterised in that** the spiral metering abutment (18) is formed at a proximal end of the metering member (15) and the other counterpart metering abutment (14) is formed by a projection protruding from the drive member (6) transversely with respect to the feed direction.

## Revendications

1. Dispositif d'administration dosée d'un produit injectable, le dispositif comprenant:
a) un logement (1), avec un réservoir (2) pour le produit,
b) un piston (3) qui repousse le produit hors du réservoir (2) lors d'un déplacement dans une direction d'avancement sur une sortie de réservoir (4),
c) un élément d'actionnement (6) qui peut coulisser le long d'un axe de déplacement (V) dans la direction d'avancement pour atteindre une position finale proximale et en sens inverse de la direction d'avancement pour atteindre une position finale distale,
d) un élément de retrait/sortie (5) dont le déplacement en sens inverse de la direction d'avancement est empêché et qui est entraîné par l'élément d'actionnement (6) lors d'un déplacement de celui-ci dans la direction d'avancement et qui déplace par ailleurs le piston (3) dans la direction d'avancement,
e) un élément de dosage (15) qui peut être pivoté autour de l'axe de déplacement (V) de l'élément d'actionnement (6) pour régler la dose de produit qui peut être distribuée lors d'une administration,
f) sachant que, dans la position finale distale, l'élément d'actionnement (6) et l'élément de dosage (15) viennent en butée chacun avec au moins une butée de dosage (14, 18) dont l'une est formée sur l'élément d'actionnement (6) et l'autre sur l'élément de dosage (15) et sachant que
g) au moins une de ces butées de dosage (14, 18) tourne au moins en partie en spirale autour de l'axe de déplacement (V) de l'élément d'actionnement (6), **caractérisée en ce que**
h) la au moins une butée de dosage (18) en spirale comprend un cours continu avec une pente (α) constant.

2. Dispositif selon la revendication 1, **caractérisé en ce que** l'élément de dosage (15) peut être pivoté entre des positions d'angle de rotation spécifiques ou définissables, de préférence en l'enclenchant dans les positions crantées de l'angle de rotation, et **en ce que** l'autre butée de dosage (14) formée de préférence sur l'élément d'actionnement (6) s'étire jusqu'à la butée de dosage (18) en spirale formée de préférence sur l'élément de dosage (15) selon un angle qui est supérieur à un espacement angulaire entre les positions d'angle de rotation directement consécutives de l'élément de dosage (15).

3. Dispositif selon la revendication précédente, **caractérisé en ce que** l'autre butée de dosage (14) s'étire selon un angle qui est égal au moins au double de l'espacement angulaire entre les positions d'angle de rotation directement consécutives de l'élément de dosage (15).

4. Dispositif selon l'une quelconque des revendications 2 ou 3, **caractérise en ce que** l'autre butée de dosage (14) s'étire scion un angle qui est égal au maximum à dix fois l'espacement angulaire entre les positions d'angle de rotation directement consécutives de l'élément de dosage (15).

5. Dispositif selon l'une quelconque des revendications précédentes, **caractérisé en ce que** l'autre élément de dosage (14) conçu de préférence sur l'élément d'actionnement (6) comprend la même pente (α) que la butée de dosage en spirale (18) jusqu'à la butée de dosage en spirale (18) formée de préférence sur l'élément de dosage (15).

6. Dispositif selon la revendication précédente, **caractérisé en ce que** la butée de dosage en spirale (18) est formée sur la face proximale de l'élément de dosage (15) et **en ce que** l'autre élément de dosage (14) est formé à travers une came faisant saillie de façon transversale par rapport à la direction d'avancement de l'élément d'actionnement (6).
